# EUROPEAN PATENT SPECIFICATION

(11) **EP 0 452 179 B1**
(45) Date of publication and mention of the grant of the patent: **12.06.1996**
(21) Application number: 91400815.6
(22) Date of filing: 26.03.1991
(51) Int. Cl.: A61K 47/48

(54) **Polymer-combined drug for gastric treatment and a method for producing the drug**
Polymerkombiniertes Arzneimittel zur Magenbehandlung und Verfahren zu dessen Herstellung
Médicament combiné avec un polymère pour le traitement gastrique et son procédé de préparation

(30) Priority: 28.03.1990 JP 77068/90; 09.07.1990 JP 179691/90
(43) Date of publication of application: 16.10.1991
(73) Proprietor: NIPPON OIL AND FATS COMPANY, LIMITED, Chiyoda-ku Tokyo (JP); Koyama, Yoshiyuki, Noda-shi, Chiba-ken (JP); Kojima, Shuji, Kashiwa-shi, Chiba-ken (JP)
(72) Inventor: Koyama, Yoshiyuki, Noda-shi, Chiba-Ken (JP); Kojima, Shuji, Kashiwa-shi, Chiba-Ken (JP); Miyazaki, Tsuyoshi, Tsukuba-shi, Ibaraki-Ken (JP); Suginaka, Akinori, Chigasaki-shi, Kanagawa-Ken (JP); Matsumoto, Takeo, Tsukuba-shi, Ibaraki-Ken (JP); Murata, Yoshishige, Tsukuba-shi, Ibaraki-Ken (JP)
(74) Representative: Moncheny, Michel

(56) References cited:
- EP-A- 0 077 529
- EP-A- 0 187 547
- EP-A- 0 397 307
- JOURNAL OF BIOACTIVE AND COMPATIBLE POLYMERS, vol. 5, January 1990, pages 53-64, Technomic Publishing Co., Inc., Lancaster, PA., US; E. YASHIMA et al.:"Polymer drugs and polymeric drugs, Part VII: Antitumor conjugated polymeric drugs consisting of 5-fluorouracil and polyanionic polymers"
- J. MACROMOL. SCI.-CHEM., vol. A24, no. 9, 1987, pages 1011-1032, Marcel Dekker,Inc., New York, US; T. OUCHI et al.: "Syntheses of 5-fluorouracil-terminatedmonomethoxypoly(ethylene glycol)s, their hydrolysis behavior, and theirantitumor activities"
- CHEMICAL ABSTRACTS, vol. 100, no. 24, June 1984, page 357, abstract no.197692q, Columbus, Ohio, US; S. ZALIPSKY et al.: "Attachment of drugs topolyethylene glycols"
- CHEMICAL ABSTRACTS, vol. 98, no. 25, June 1983, page 127, abstract no. 210586v,Columbus, Ohio, US; C. JOHANSSON et al.: "Stimulation of gastric bicarbonatesecretion by E2 prostaglandins in man"
- PATENT ABSTRACTS OF JAPAN, vol. 5, no. 204 (C-85)(876), 24th December 1981; & JP-A-56 123 917
- PHARMACEUTICAL RESEARCH, vol. 7, no. 8, August 1990, pages 863-868, Plenum Publishing Corporation, Stuttgart,DE; M.D. DONOVAN et al.: "Absorption ofpolyethylene glycols 600 through 2000: The molecular weight dependence ofgastrointestinal and nasal absorption"

## Description

The present invention relates to the use of a conjugate resulting from the bonding of a poly(oxyalkylene) polymer or copolymer to a medicine, said conjugate having directional characteristics to digestive organs by which the drug is specifically carried into the stomach.

Hitherto, for carrying medicines having directional characteristics to the stomach or intestines, drugs such as an oral drug and a suppository should be administered to the internal organs directly or adjacently. When a medicine is administered intravenously, since the blood concentration is increased rapidly, the secondary effect of the medicine causes trouble. Further, since the medicine is excreted rapidly, there are problems such as low durability of the effect of the medicine and the like.

In polyalkyleneoxy compounds, particularly polyethylene glycol has low toxicity and non-immunogenicity. It is known widely that the compound administered orally or intravenously is little harmful for the human body. However, the movement of these compounds in the body after medication is little reported, and it is reported only that the compounds are rapidly excreted after intravenous injections.

In general, it is said that water-soluble materials having relatively high molecular weight are bad for permeability to a mucosa and they are little absorbed (M. D. Donovan et al, Pharmaceutical Res., Vol. 7, 863 (1990)).

As a mucosa of the digestive tract prevents absorption of water-soluble medicines which are orally administered, it is a serious obstacle to take effect by using various water-soluble medicines, such as ionic cholinolytics and peptide medicines.

J. Macromol. Sci.-Chem. A 24(9), 1011-1032, (1987) is concerned with the preparation of antitumor drugs consisting of biologically active 5-fluorouracil attached to a polyethylene glycol derivative.

Eur. Polym. J. 19, 1177-1183 (1983), describes the combination of polyethylene glycol with several kinds of medicines such as aspirin, amphetamine, quinidine and atropine.

However, none of these documents suggests the accumulation of the resulting drug in the stomach upon administration thereof.

The present invention aims to solve the above problems by use of a conjugate resulting from the bonding of a poly(oxyalkylene) polymer or copolymer to a medicine, said conjugate having directional characteristics to the stomach by which a medicine, for example a therapeutic drug or a diagnostic drug, is administered by an intravenous injection so that it can be specifically carried into the stomach, and by which it is specifically and efficiently absorbed from the stomach by oral administration.

The inventors of the present invention have found that a compound combined with a medicine and a polymer having an alkyleneoxy group, such as polyethylene glycol and the like, as a principal repeating unit shows the following polymer effects by the polymerization of the medicine itself :
(1) the secondary effect and the like are diminished by avoidance of undesirable accumulation of the medicine into the internal organs, tissues and the like, and
(2) the effect of the medicine are maintained by delay of excretion, and it has the following characteristics which is not realized by known medicines and carriers :
(3) the compound has a property of very high directional characteristics to the stomach,
(4) the compound is permeable into gastric mucosa to be secreted into the stomach,
(5) the stomach acts as a reservoir of the medicine, and
(6) the compound is rapidly absorbed by oral administration into the intestines and it is efficiently absorbed from the stomach by passing rapidly through a gastric mucosal barrier. Then, the present invention has been completed,
Namely, the present invention is directed to a conjugate resulting from the bonding of a poly(oxyalkylene)polymer or copolymer to a medicine, said conjugate having directional characteristics to the stomach, characterized in that it comprises a medicine combined with a polymer which has an alkyleneoxy group as a repeating unit. According to the present invention the conjugate is produced by reacting a polyoxy alkylene glycol having one or more terminal functional groups with a medicine, if necessary in the presence of a catalyst and in a solvent.

The following description illustrates the present invention more specifically.

As the polymer having an alkyleneoxy group as a repeating unit, polyethylene glycol, polypropylene oxides, polybutylene oxides, ethylene oxide-propylene oxide copolymers, polymers obtained by substituting the terminal groups of the above polymers with an acyl, amino or allyl group, and copolymers of the above polymers and acrylic acid, maleic acid, styrene or the like are preferably exemplified. The molecular weight of the above polymer is 100-200,000, preferably 800-100,000.

The above-mentioned polymers having desired molecular weight can be obtained by addition polymerization of ethylene oxide, propylene oxide or the like by using a well-known method.

From these polymers, the drugs having directional characteristics to the stomach are obtained by binding medicines such as a therapeutic drug and a diagnostic drug.

It is enough to use any compound as the therapeutic drug, preferably drugs which take effect directly on diseases of the stomach.

More preferably the drug is selected from cimetidine, famotidine, prostaglandin E₁, prostaglandin E₂ and dimethyl prostaglandin E₂.

As the diagnostic drugs, radioisotopelabeled polymers having an alkyleneoxy group as a principal repeating unit can be used. To obtain the polymers, a method for ¹²⁵I labeling a polymer by chloramine T or glucose oxidase, a method for ¹²⁵I labeling a polymer after tyrosine is introduced into the polymer, a method for bonding covalently a ¹²⁵I labeled compound or metal chelate to a polymer and the like can be used.

Further, any methods for combining medicines with the above polymers including covalent bond, ionic bond, coordinate bond, Schiff base formation and the like can be used and the most suitable method can be selected according to each medicine. Then, functional groups required to carry the medicine in the polymer is preferably introduced into the polymer. The following methods are exemplified;
1) a method for reacting PGE, in which a terminal carboxy group is introduced, with a medicine having an amino group and/or a hydroxyl group and/or thiol group in the molecule,
2) a method for reacting PGE, in which an amino group and/or a hydroxyl group and/or a thiol group at the terminal is introduced, with the above medicine,
3) a method for reacting a copolymer, which is obtained from PGE having a terminal allyl group and carboxylic acid anhydride to have an carboxyl group, with the above medicine.

In these reactions, triethylamine, pyridine, sodium hydroxide, potassium hydroxide, sodium carbonate, potassium carbonate and N, N'-dicyclohexyl carbodiimide, as a catalyst or a condensation agent, and activated esterification agents such as succinimides and nitrophenol can be used. When necessary, water,benzene, toluene, ether, acetone, ethyl acetate, dimethylformamide, dimethyl sulfoxide and the like can be used as a solvent. The reaction temperatures are -10°C to +200°C, preferably 0 to 120°C, and the reaction times are one minute to 170 hours, preferably 10 minutes to 24 hours.

According to the invention, the conjugate can be formed into various forms. For example, tablets, powder, granules, aqueous solutions, suspensions, emulsions. capsules, medicinal oil, ointment and suppositories are preferred. The polymer-combined drug of the present invention can be administered by injection, oral, sublingual administration or suppository and the dosage can be determined optionally within the limits of conventional use.

When the conjugate which has directional characteristics to the stomach is used, therapeutic drugs or diagnostic drugs can be selectively carried by an intravenous injection into the stomach. Accordingly, the greatest effect of the therapeutic drugs or diagnostic drugs is provided in the stomach and the secondary effect can be diminished in the other internal organs.

When the conjugate is administered orally or by a suppository from the rectum, the concentration in blood can be stably maintained for a long time in comparison with conventional methods, and the conjugate can be absorbed or taken in the stomach directly and efficiently.

Since the drug is directly absorbed from the stomach, adsorption and decomposition in the liver which are problems in the absorption through the small intestines can be avoided.

Further, according to the method of the present invention, the aimed conjugate can be obtained certainly and efficiently.

The following examples illustrate the present invention more specifically.

### Example 1

One gramme of polyethylene glycol having terminal carboxyl groups (molecular weight: 3020) was dissolved in 14 ml of dry acetone. 137 mg of famotidine and 0.5 ml of dimethyl sulfoxide were added to the solution to dissolve thoroughly. Then, 0.24 ml of triethylamine and 162 mg of N-N'-dicyclohexyl carbodiimide were added to the solution in an ice bath, and the mixture was reacted at 5°C for 12 hours. After the reaction was finished, the solvent was removed under reduced pressure. The residue was dissolved in a little water and the solution was gel-filtered with Sephadex G-25. The high molecular weight fraction was lyophilized to obtain a white spongy polymer-combined drug (yield: 974 mg). Famotidine contained in the drug was 3.8% by weight as a result of elemental analysis.

The constitutional formula is shown: 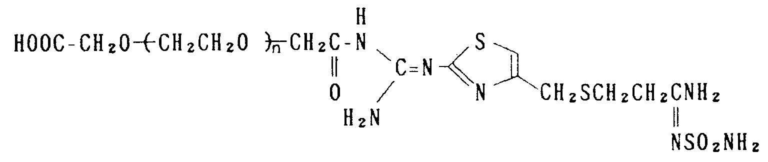
IR (KBr, cm⁻¹) : 1700 (CO), 1300, 1150 (SO₂NH₂) UV : λₘₐₓ 305 nm

### Example 2

402 mg of an alternating copolymer of polyethylene glycol having a terminal allyl group and maleic anhydride (molecular weight: 20000) was dissolved in 4 ml of dry acetone. 0.11 ml of triethylamine, 251 mg of famotidine and 2 ml of dimethyl sulfoxide were added to the solution, and the mixture gas reacted at 5°C for 12 hours. After the reaction was finished, the solvent was removed under reduced pressure. The residue was dissolved in a little water, the solution was purified with Sephadex G-25 and it gas lyophilized to obtain a white solid polymer-combined drug (yield:429 mg). Famotidine contained in the drug was 19.9% by weight as a result of elemental analysis.

The constitutional formula is shown: 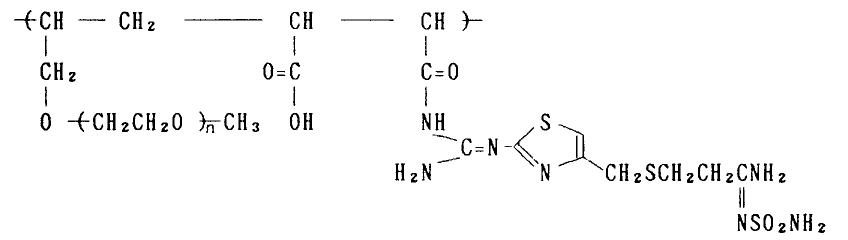
IR (KBr, cm⁻¹) : 1700 (CO), 1300, 1150 (SO₂NH₂)

### Example 3

### Synthesis of PGE having a terminal group of famotidine

Ten g of famotidine was dissolved in a mixture solvent of methylene chloride : dimethylsulfoxide (abbreviated as DMSO in the following) = 1 : 1, 3.03 gramme of triethylamine was added to the solution and the mixture was stirred for 30 minutes. To the mixture, 20 ml of the above mixture solvent in which 4.5 g succinic anhydride was dissolved was added dropwise for 30 minutes, and the mixture obtained was stirred for 12 hours at room temperature. Then, the reaction mixture was poured into 100 ml of water, the precipitate obtained was recovered by centrifugation, washed repeatedly with water and freeze-dried to obtain 10.8 g (yield:82.6%) of a yellowish white powder. One g of the powder was dissolved in 5 ml of dimethylformamide (abbreviated as DMF in the following), 0.25 ml of triethylamine and 0.6 mg of N, N'-dicyclohexyl carbodiimide were added to the solution, and the mixture was stirred for 5 hours in ice cooling. To the mixture, 10 ml of a DMF solution in which 8.5 g of polyethylene glycol having a terminal amino group (MW = 3360, manufactured by NIPPON OIL & FATS CO., LTD.) was dissolved was added dropwise for 30 minutes, and the mixture obtained was stirred for one hour in ice cooling. Further, the reaction mixture was stirred for 12 hours at room temperature and poured into 20 ml of water. After the precipitate was removed by centrifugation, the supernatant was filtered with a membrane filter having a hole diameter of 0.45 µm and the filtrate was gel-filtered with a Sephadex G-25. The filtrate purified was freeze-dried and 7.2 g of white powder drug was obtained. Famotidine contained in the drug was 6.5 % by weight from the result of elemental analysis.

The constitutional formula is shown: 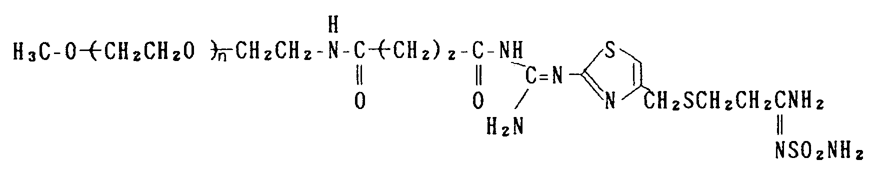
IR (KBr, cm⁻¹) : 1700 (CO), 1300, 1150 (SO₂NH₂)
UV : λₘₐₓ 305 nm.

### Example 4

The same procedure as in Example 3 was repeated except that cimetidine was used as a medicine to synthesize a polymer-combined drug. 7.1 g of white powder was obtained. In the drug, the cimetidine content was 6.2 % by weight as a result of elemental analysis.

The constitutional formula is shown: 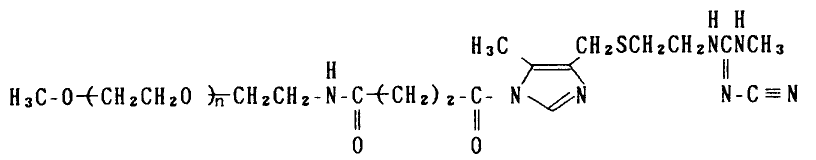
IR (KBr, cm⁻¹) : 2250 (CN), 1670 (CON<), 1700 (CO)

### Example 5

The same procedure as in Example 2 was repeated except that cimetidine was used as a medicine to synthesize a polymer-combined drug. 544 mg of a white powder sample was obtained. In the drug, the cimetidine content was 18.8% by weight as a result of elemental analysis.

The constitutional formula is shown: 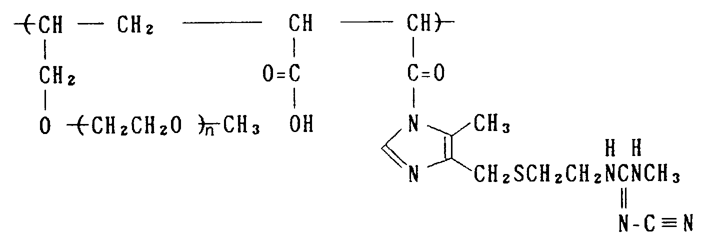
IR (KBr, cm⁻¹) : 2250 (CN), 1670 (CON<), 1690 (COOH)

### Example 6

Using the polymer-combined drug obtained in Example 3, the inhibition of gastric secretion was determined by the following method.

Abdomen of a rat (six weeks, 159 g) was operated in urethane narcosis, a cannula was inserted in the pylorus. The stomach was washed three times with an isotonic sodium chloride solution and 3 ml of an isotonic sodium chloride solution was filled in the stomach. The solution was taken out after one hour and 3 ml of a new isotonic sodium chloride solution was filled in the stomach.

Histamine hydrochloride was intravenously injected into mouse tails in the ratio of 0.6 mg/animal every one hour, and after two hours, famotidine alone or the polymer-combined drug (famotidine content: 0.45 mg/animal) was intravenously injected.

3.5, 4 and 4.5 hours after the drugs were administered, the inhibition of gastric secretion was determined. The results are shown in Table 9.

**Table 9**

| | 3.5 hours | 4.0 hours | 4.5 hours |
|---|---|---|---|
| Famotidine | 37 % | 33 % | 36 % |
| Polymer drug | 46 | 61 | 53 |

Further, the intravenous injection method was changed to a direct administration method into the duodenum, and the inhibition of gastric secretion was determined. The results obtained 1.5, 2.5 and 3.5 hours after the drugs were administered, are shown in Table 10.

**Table 10**

| | 1.5 hours | 2.5 hours | 3.5 hours |
|---|---|---|---|
| Famotidine | 72 % | 16 % | 7 % |
| Polymer drug | 37 | 36 | 31 |

As shown in the results, the effect of the polymer-combined drug is superior to that of famotidine alone with the exception of a part, and it is found that the polymer combined drug has durability.

### Example 7

The polymer-combined drug obtained in Example 5 was intravenously injected into mouse tails, the inhibition of gastric sescretion was examined by the following method.

As soon as pylorus of a rat (150 mg) was ligated, an isotonic sodium chloride solution as a control, the polymer-combined drug obtained in Example 4 (cimetidine content: 0.4 mg) and 0.4 mg of cimetidine were administered, respectively, and then 0.6 mg of histamine hydrochloride was intravenously injected, the gastric juice was taken out after one hour, and content of acid was determined.

As a result, in comparison with the control, the inhibition effect of gastric secretion of cimetidine was 22 %, and that of polymer-combined drug was 41 %. Accordingly, it is found that the effect of the polymer-combined drug is superior to that of cimetidine alone, because the polymer-combined drug accumulates selectively in the stomach.

### Example 8

Using the polymer-combined drugs obtained in Examples 4 and 5, the inhibition of gastric secretion were determined by the following method.

Abdomen of male rats (180-240 g) were used after one night fasting. In urethane narcosis, cannulas for refluxing a liquid in the stomach were inserted and fixed in the pylorus and the cardiac orifice. A liquid for refluxing in the stomach was injected through the cardiac orifice canule by using a continuous injection pump (0.5 ml/minute), and the liquid was recovered through the pylorus canule.

Further, histamine hydrochloride (4.0 mg/kg/hour) was injected through the tail vein by using a continuous injection pump to stimulate the secretion of the gastric juice.

The reflux liquid flowing from the pylorus canule was recovered each 30 minutes and titrated with an automatic titrator until the pH value attained to 7.0, and the secretion quantity of the gastric juice was determined. The samples were intravenously administered in the tails each 1.5 hours after the injection of histamine hydrochloride was begun.

0.5, 1.5, 2.5, 3.5 and 4.5 hours after the samples of cimetidine, PEG-CIM 12 (exp. 12) and LPM-CIM 13 (Example 13) were administered, the inhibition of gastric secretion was determined. The results are shown in Table 11.

**Table 11**

| | 0.5 | 1.5 | 2.5 | 3.5 | 4.5 | (hours) |
|---|---|---|---|---|---|---|
| Cimetidine | 30.7 | 28.6 | 32.4 | 21.1 | 24.4 | (%) |
| PEG-CIM 12 | 24.7 | 38.5 | 43.4 | 38.9 | 24.1 | |
| LPM-CIM 13 | 43.6 | 33.8 | 39.5 | 36.7 | 37.0 | |

In each case, 3.0 mg/kg of cimetidine content was intravenously injected.

### Example 9

Using the conjugate obtained in Examples 1 and 2, the inhibition of gastric secretion were determined in the same method as shown in Example 7.

0.5, 1.5, 2.5, 3.5 and 4.5 hours after the samples of famotidine, PEG-FAM 9 which is a polymer-combined drug of famotidine (Example 9) and LPM-FAM 10 (Example 10) were administered, the inhibition of gastric secretion was determined. The results are shown in Table 12.

**Table 12**

| | 0.5 | 1.5 | 2.5 | 3.5 | 4.5 | (hours) |
|---|---|---|---|---|---|---|
| Famotidine | 45.2 | 71.6 | 21.1 | -9.2 | -25.3 | (%) |
| PEG-FAM 9 | 40.0 | 72.6 | 34.8 | 30.6 | 4.6 | |
| LPM-FAM 10 | 48.9 | 73.9 | 50.9 | 42.0 | 15.6 | |

In each case, 0.1 mg/kg of famotidine content was intravenously injected.

As shown in the results of Examples 6-9, the effect of the conjugate is superior to that of cimetidine or famotidine alone, and it is found that the conjugate has durability, particularly in the case of the polymer-combined drug of famotidine, the durability is remarkably prolonged.

### Example 10

200 mg of PEG having a terminal methoxy group and a terminal amino group (MW = 4300, manufactured by NIPPON OIL & FATS CO., LTD.) and 100 mg of chloromethylbenzoic acid were dissolved in 3 ml of methylene chloride. To the solution, N, N'-dicyclohexyl carbodiimide were added, and the mixture was stirred for 4 hours at room temperature and was permitted to stand for 12 hours. The reaction mixture was filtered, and the filtrate was concentrated under reduced pressure. The residue obtained was dissolved in 2 ml of water, the solution was gel-filtered with Sephadex G-25, and the polymer fraction was freeze-dried. White powder of PEG (PEG-CB) having a terminal chloromethylphenyl group was obtained. Water was separated as the benzene azeotrope from 20 mg of the powder obtained. Further the compound was dried in vacua in the presence of phosphorus pentaoxide and dissolved in 0.1 ml of dimethylformamide (A solution).

The other hand, 2 mg of 16, 16-dimethylprostaglandine E₂ was dissolved in 0.2 ml of a mixture solvent of ethanol : ethyl acetate = 9 : 1, the solution was added to 4 ml of 50 % of a cesium carbonate aqueous solution and the mixture was stirred for 30 minutes. The reactant was freeze-dried to obtain cesium 16, 16-dimethylprostaglandine. The whole of the compound was added to the above A solution, and the mixture was stirred for 18 hours at room temperature and concentrated under reduced pressure. Then, the residue was dissolved in 2 ml of water, and the solution was gel-filtered with Sephadex-25 to obtain a high molecular fraction. The fraction was freeze-dried and 20 mg of white powder of PEG-PG-A.

Further, from the difference of the absorbance at 270 nm between PEG-CM and PEG-PG, the content of 16, 16-dimethylprostaglandine E₂ in PEG-PG-A was 0.38 % by weight.

### Example 11

0.75 mg of PEG having a terminal methoxy group and a terminal amino group (MW = 4300, manufactured by NIPPON OIL & FATS CO., LTD.), 0.5 mg of prostaglandin E₂, 0.16 mg of triethylamine and 20 mg of N, N'-dicyclohexyl carbodiimide were added in 0.5 ml of ethanol, and the mixture was stirred at room temperature for 18 hours and the reactant was concentrated under reduced pressure. The residue obtained was dissolved in 1 ml of water, the solution was gel-filtered with Sephadex G-25 to obtain a high molecular fraction, and the fraction was freeze-dried. 4.1 mg of white powder of PEG-PG-B was obtained. The content of prostaglandin E₂ was 0.22 % by weight.

### Example 12

0.5 mg of prostaglandin E₁ and 2.1 mg of N, N'-carbonyldiimidazole were added to 1 ml of dried tetrahydrofuran, and the mixture was stirred for 15 minutes at room temperature. To the mixture, 1 ml of a toluene solution of PEG (MW = 4300, manufactured by NIPPON OIL & FATS CO., LTD.) having a terminal methoxy group and a terminal hydroxy group which was dried by azeotropic distillation (6.7 mg/ml) was added. The mixture was stirred for 72 hours at room temperature and the reactant was concentrated under reduced pressure. The residue was dissolved in 1 ml of water, the solution was gel-filtered with Sephadex G-25 to obtain a high molecular fraction, and the fraction was freeze-dried. 3.9 mg of white powder of PEG-PG-C was obtained. The content of prostaglandin E₁ was 0.33 % by weight.

### Example 13

0.5 mg of 16, 16-dimethylprostaglandin E₂ and 2.1 mg of N, N'-carbonyldiimidazole were added to 1 ml of dried tetrahydrofuran, and the mixture was stirred for 15 minutes at room temperature. To the mixture, 1 ml of a toluene solution of PEG (MW = 2000, manufactured by NIPPON OIL & FATS CO., LTD.) having two terminal hydroxy groups which was dried by azeotropic distillation (31.2 mg/ml) was added. The mixture was stirred for 72 hours at room temperature and the reactant was concentrated under reduced pressure. The residue was dissolved in 1 ml of water, the solution was gel-filtered with Sephadex G-25 to obtain a high molecular fraction, and the fraction was freeze-dried to obtain 29.1 mg of white powder of PEG-PG-D. The content of 16, 16-dimethylprostaglandin E₂ was 0.043 % by weight.

### Example 14

0.2 ml of a physiological sodium chloride solution as a control and 0.2 ml of a physiological sodium chloride solution of PEG-PG-A obtained in Example 18 (0.39 mg/ml) were orally administered to mice of 8 weeks (ddY, male, about 30 g, each n = 3). After 30 minutes, 0.2 ml of pure ethanol was orally administered. After 50 minutes the mice were killed in a syncopic state and vivisected, and the damage of the stomach was examined. As a result, 5 to 7 spots of big hemorrhagic ulcer were observed in individuals of the control. However, one spot of small hemorrhage was observed in individuals of PEG-PG-A administration group.

### Example 15

The same procedure as in Example 14 was repeated except that a physiological sodium chloride solution of PEG-PG-D (2 mg/ml) obtained in Example 13 was used instead of PEG-PG-A, and the damage of the stomach was examined. As a result, 5 to 7 spots of big hemorrhagic ulcer were observed in individuals of the control. However, one spot of small hemorrhage was observed in individuals of the PEG-PG-D administration group.

### Example 16

0.2 ml of a physiological sodium chloride solution as a control and 0.2 ml of a physiological sodium chloride solution of PEG-PG-A obtained in Example 10 (39 µg/ml) were intravenously injected into mice tails of 8 weeks (ddY, male, about 30 g, each n = 3). After 2 hours, 0.2 ml of pure ethanol was orally administered. After 50 minutes the mice were killed in a syncopic state and vivisected, and the damage of the stomach was examined. As a result, 5 to 7 spots of big hemorrhagic ulcer were observed in individuals of the control. However, zero or one spot of small hemorrhage was observed in individuals of the PEG-PG-A administration group.

### Example 17

0.2 ml of a physiological sodium chloride solution as a control and 0.2 ml of a physiological sodium chloride solution of PEG-PG-B obtained in Example 11 (68 µg/ml) were intravenously injected into mice tails of 8 weeks (ddY, male, about 30 g, each n = 3). After 2 hours, 0.2 ml of pure ethanol was orally administered. After 50 minutes the mice were killed in a syncopic state and vivisected, and the damage of the stomach was examined. As a result, 5 to 7 spots of big hemorrhagic ulcer were observed in individuals of the control. However, zero or one spot of small hemorrhage was observed in individuals of the PEG-PG-B administration group.

### Example 18

0.2 ml of a physiological sodium chloride solution as a control and 0.2 ml of a physiological sodium chloride solution of PEG-PG-C obtained in Example 12 (45 µg/ml) were intravenously injected into mice tails of 8 weeks (ddY, male, about 30 g, each n = 3). After 2 hours, 0.2 ml of pure ethanol was orally administered. After 50 minutes the mice were killed in a syncopic state and vivisected, and the damage of the stomach was examined. As a result, 5 to 7 spots of big hemorrhagic ulcer were observed in individuals of the control. However, one or two spots of small hemorrhage was observed in individuals of the PEG-PG-C administration group.

### Example 19

0.2 ml of a physiological sodium chloride solution as a control and 0.2 ml of a physiological sodium chloride solution of PEG-PG-D obtained in Example 13 (200 µg/ml) were intravenously injected into mice tails of 8 weeks (ddY, male, about 30 g, each n = 3). After 2 hours, 0.2 ml of pure ethanol was orally administered. After 50 minutes the mouse was killed in a syncopic state and vivisected, and the damage of the stomach was examined. As a result, 5 to 7 spots of big hemorrhagic ulcer were observed in individuals of the control. However, one spot of small hemorrhage was observed in individuals of the PEG-PG-D administration group.

As shown in the results of Examples 14-19, the anti-ulcer effect of PEG-prostaglandin in the stomach was certainly acknowledged.

## Claims

1. Use of a conjugate resulting from the bonding of a poly(oxyalkylene)polymer or copolymer to a medicine, said polymer or copolymer having a molecular weight from 800 to 100000 and carrying optional terminal functional groups, for the manufacture of a medicament for the treatment or diagnosis of the stomach, and said medicine being selected from cimetidine, famotidine, prostaglandin E₁, prostaglandin E₂ and dimethyl prostaglandin E₂.

2. Use according to claim 1, wherein the temrinal functional group of said polymer or copolymer is selected from carboxyl, hydroxyl, amino and allyl.

3. Use according to any of claims 1 and 2, wherein said polymer or copolymer is labeled with a radioactive iodine.

4. Use according to any of claims 1 to 3, wherein said polymer is polyethylene glycol.

5. Use according to claim 1, wherein said copolymer is a copolymer of polyethylene glycol carrying an allyl group and maleic anhydride as terminal functional groups.

## Patentansprüche

1. Verwendung eines Konjugats, das durch die Bindung eines Poly(oxyalkylen)-Polymers oder -Copolymers an ein Medikament entsteht, wobei das Polymer oder Copolymer ein Molekulargewicht von 800 bis 100000 aufweist und wahlfreie endständige funktionelle Gruppen trägt, für die Herstellung eines Arzneimittels für die Behandlung oder Diagnose des Magens, wobei das Medikament aus Cimetidin, Famotidin, Prostaglandin E₁, Prostaglandin E₂ und Dimethylprostaglandin E₂ ausgewählt ist.

2. Verwendung nach Anspruch 1, wobei die endständige funktionelle Gruppe des Polymers oder Copolymers aus Carboxyl, Hydroxyl, Amino und Allyl ausgewählt ist.

3. Verwendung nach einem der Ansprüche 1 und 2, wobei das Polymer oder Copolymer mit radioaktivem Iod markiert ist.

4. Verwendung nach einem der Ansprüche 1 bis 3, wobei das Polymer Polyethylenglycol ist.

5. Verwendung nach Anspruch 1, wobei das Copolymer ein Copolymer von Polyethylenglycol ist, das als endständige funktionelle Gruppen eine Allylgruppe und Maleinsäureanhydrid trägt.

## Revendications

1. Utilisation d'un conjugué résultant de la liaison d'un polymère ou d'un copolymère de poly(oxyalkylène) à un médicament, ledit polymère ou copolymère possédant un poids moléculaire de 800 à 100.000 et portant éventuellement des groupes fonctionnels à ses extrémités, dans la fabrication d'un médicament destiné au traitement ou au diagnostic gastrique, ledit médicament étant choisi parmi la cimétidine, la famotidine, la prostaglandine E₁, la prostaglandine E₂ et la diméthylprostaglandine E₂.

2. Utilisation selon la revendication 1, dans laquelle le groupe fonctionnel terminal dudit polymère ou copolymère est choisi parmi les groupes carboxyle, hydroxyle, amino et allyle.

3. Utilisation selon l'une des revendications 1 et 2, dans laquelle ledit polymère ou copolymère est marqué avec de l'iode radioactif.

4. Utilisation selon l'une des revendications 1 à 3, dans laquelle le dit polymère est le polyéthylène glycol.

5. Utilisation selon la revendication 1, dans laquelle ledit copolymère est un copolymère de polyéthylène glycol portant un groupe allyle et un anhydride maléique en tant que groupes fonctionnels terminaux.
